# EUROPEAN PATENT APPLICATION

(11) **EP 4 697 342 A1**
(43) Date of publication of application: **18.02.2026**
(21) Application number: 24195077.3
(22) Date of filing: 17.08.2024
(51) Int. Cl.: G16H 20/30

(54) **DYNAMIC PHYSICAL ACTIVITY RATING DETERMINING SYSTEM USING USER PROGRAMMED FUNCTIONS**

(71) Applicant: Bajer, Maciej, 01-864 Warsaw Mazowieckie (PL)
(72) Inventor: Bajer, Maciej, 01-864 Warsaw Mazowieckie (PL)

(57) **Abstract**

The invention relates to a computer system, which calculates personalized rating for a physical activity, based on multi-domain parameters and user programmed functions. System comprises user device with client application, a server with server application, and a connection between user device and a server. System uses user programmed fitness configurations and user programmed fitness events to process physical activity analysis. Fitness configuration is a set of conditions and rules which is used for every day usage. Fitness event is a set of conditions and rules which is used for specific, activated moment using multi-domain activation factor. System assists user in making decisions about any physical activity and managing physical activity sessions. This invention relates generally to information engineering and physical activity.

## Description

### FIELD OF INVENTION

The invention relates to a computer system, in a computer network, which calculates personalized rating for a physical activity, based on multi-domain parameters and user programmed functions. System assists user in making decisions about any physical activity and managing physical activity sessions. This invention relates generally to information engineering and physical activity.

### BACKGROUND OF INVENTION

Physical activity is necessary to keep body in shape. Physical activity rating may be an effective way to describe usefulness and quality of an activity. It may have big impact on health or life, depending on the health condition of a person.

The earliest activity ratings were effective for their simplicity, but as technology progressed ratings remained unchanged for modern solutions. Today it is common to find description about energy to burn, distance to run or weight to lift on many physical trainings and exercises. Such ratings doesn't include detailed information about activity.

A person who want to try new activity often doesn't know about the impact the activity may have on their body and health. Before trying new activity, it remains unknown how useful such activity is. This causes that people are not trying many new exercises or trainings, but they keep doing already familiar activities repetitively. This can lead to many problems such as uneven body development, injury, monotony or other condition. Because of these problems, many people eventually give up physical activity.

Without dynamic generated activity ratings, it is required to get to know new activity and mark it with own rating manually, repeating for each activity respectively. However such rating would be valid only for this person who made the evaluation. For other people specific activity could have other rating, because people may have completely different physical needs or health issues.

For example US11423281 B2 relates to recommending physical activities for user profile data, using machine-learning model. It lacks analyzing objective activity data (e.g. required equipment for an activity, required health condition, body parts used in activity), subjective activity data (e.g. difficulty level), and non-activity data (e.g. time of analysis, location of analysis, history of user's activity, user state of mind). Moreover it also evaluates activity in a predefined way, based on a trained machine-learning model, which cannot be changed to personalize activity ratings for each user individually.

There is need to use personalized set of rules to generate personalized physical activity rating. Moreover there is need to use temporary set of rules depending of personalized factors (e.g. different rules for specific time interval or specific location).

There is no solution that process physical activity details to generate rating describing activity. Current physical activities descriptions are too generic and shallow. They lack a lot of details, depth and personalization which is defined by a unique life of every person.

### SUMMARY

The invention is defined by the appended independent claims. Additional features, uses and advantages of the concepts disclosed herein are set forth in the description which follows, and in part will be obvious from the description, or may be learned by practice of the described technologies.

The present disclosure describes system for dynamically calculating physical activity rating. System comprises a user device, a server, and a network connection between the user device and the server enabling data transfer between them. The user device runs a client application. The server runs a server application.

The present disclosure includes 2 analysis processing methods based on the device type: the user device and a server.

The present disclosure describes 2 analysis types depending on object with user programmed functions: fitness configuration or fitness event. Both of them can be created and updated by the user. Both of them comprises sets of conditions and rules for analysis processing. Additionally fitness event contains activation factor or factors to make this fitness event activated and presented to user.

The statically-generated rating for a given physical activity does not take into account the impact on human health, the time (moment) of the physical activity, used equipment, used body parts and the impact on active body parts:
- The advantage of the invention is that it allows to determine the personalized quality rating of a physical activity for a person in an automated manner.
- The advantage of the invention is that it allows to process different physical activity attributes in the physical activity analysis.
- The advantage of the invention is that it allows to process non-activity data in the physical activity analysis.
- The advantage of the invention is that it allows to include specific and personalized features in the physical activity analysis according to user programmed functions.
- The advantage of the invention is that it allows to obtain ratings of many different physical activities in parallel at the same time.
- The advantage of the invention is that it allows to take the amount of burned energy in physical activity into account in the physical activity analysis.

This allows for faster, easier and more precise monitoring of the physical activities that is crucial to keep good shape and health for every person.

In addition, the system allows you to monitor physical activity using fitness events:
- The advantage of the invention is that it allows to determine the quality of physical activity during temporary event, which has never been developed before. When the quality of physical activity is known during specific fitness event, the user can change future decisions regarding their fitness (e.g. increase the physical activity of a specific type of exercise later this day or completely eliminate the physical activity of a specific type of exercise for next week).
- The advantage of the invention is that it allows to program temporary events for complex health or fitness needs.
- The advantage of the invention is that it allows to integrate user's fitness with other aspects of life using activation factors in fitness events.

### DETAILED DESCRIPTION

The invention comprises a user device, a server, and a network connection between the user device and the server enabling data transfer between them. The user device is a computer-type device. The user device comprises a processor, input-output devices, operating memory, and persistent memory. The server comprises a processor, input-output devices, operating memory, and persistent memory. The user device runs a client application. The server runs a server application. This invention relies on processing physical activity or physical activities data and generating the result of analysis.

The user can obtain the result of physical activity in 2 possible ways:
a) client-based analysis processing - the user device downloads necessary data from server for physical activity analysis, process physical activity analysis and presents the result of the physical activity analysis.
b) server-based analysis processing - the user device triggers the physical activity analysis process on server, downloads the result of the physical activity analysis and presents it.

Moreover, the user can analyze one or many physical activity simultaneously towards 2 defined data types:
a) fitness configuration - a description of fitness independent of any activation factors.
b) fitness event - a description of fitness determined by activation factor (primarily a time trigger, but it may be another trigger such as the user's geographic location, the user's current health status, or the user's current financial status).

Both the analysis towards fitness configuration and the analysis towards fitness event is possible to concern one single physical activity or many different physical activities at the same time.

The user device runs a client application. The client application is responsible for communicating with the server in order to analyze physical activity. The client application has 2 operating modes for the following system operating modes:
a) client-based analysis result generation - mode of data downloading and local physical activity analysis. The application downloads the necessary data from the server needed to analyze the physical activity and processes the physical activity analysis locally on the user device.
b) server-based analysis result generation - mode of downloading the result of analysis. The application downloads the physical activity analysis result from the server, which is generated on the server.

The server application of the system is running on the server. The server application is responsible for communicating with the client application (user). The server application has 2 operating modes for the following system operating modes:
a) client-based analysis result generation - mode of sending data necessary to perform physical activity analysis calculations. The server application sends the necessary data for physical activity analysis to the client application. The analysis is processed locally on the user device.
b) server-based analysis result generation - mode of locally analyzing the physical activity and sending the analysis result. The application processes the analysis of the physical activity on the server and sends the result of the analysis (without providing the data necessary to perform the calculations).

Both the client application and the server application is able to store locally in the device:
- file of the fitness configuration
- file of the fitness event
- file of the physical activity description

This symmetrical design allows to use one of the 2 mentioned above system operation modes depending on the purpose of use and hardware capabilities.

The server has a database of physical activities data. Each physical activity has an identifier that allows to quickly find a file of physical activity description. Physical activities data can be stored outside server database (for example as a QR code printed on paper sheet in fitness gym). Each physical activity is described by objective parameters (such as mass of the kettlebell or distance of workout run) and subjective parameters (such as cardio workout intensity).

Fitness configuration is a file that defines the user's fitness parameters. Fitness configuration consists of a list of multiple sets of sentences that comprehensively define the user's health and fitness. Fitness configuration has 2 types of sentences:
a) regulation - a set of sentences that generates the component result of the analysis.
b) toleration - a set of sentences that decides which regulations and tolerances are to be active and which are to be included in the final physical activity analysis result.

The regulation consists of 2 subsets:
a) logical conditions - it is a set of logical sentences connected by logical connectors, where the number of logical sentences can be 0 (the empty set can occur).
b) logical rules - it is a set of logical sentences connected by logical connectors, where the number of logical sentences must be greater than 0 (the empty set cannot occur).

In addition, the regulation has defined intermediate values for each possible outcome:
a) success - the conditions and the rules are fulfilled.
b) fail - conditions are fulfilled and the rules are not fulfilled.
c) undetermined - the physical activity does not have the data necessary to check the fulfillment of conditions or rules.

The defined intermediate values can be edited and set by the user to any other values. The intermediate values are used to get final result of the physical activity analysis. By default, the following intermediate values can be assumed:
a) success = +1
b) fail = -1
c) undetermined = 0

The toleration consists of 2 subsets:
a) logical conditions - it is a set of logical sentences connected by logical connectors, where the number of logical sentences must be greater than 0 (the empty set cannot occur).
b) references to regulations and tolerances - it is a list of regulation and tolerance identifiers that are to be canceled and excluded from the final physical activity analysis result if the current tolerance conditions are fulfilled.

A logical sentence (both in the set of logical conditions and logical rules) is the smallest unit of calculation in the analysis process. The logical sentence consists of 3 elements:
a) subject - a feature that is the subject of analysis in a logical sentence.
b) operation - mathematical operation between an subject and a value.
c) value - a defined simple value or a set of simple values.

Examples of physical activity features that can be a subject in a logical sentence are:
a) mass of the dumbbell
b) distance of the run
c) average burned energy during 60 seconds of the activity (workout)

Examples of features not related to physical activity that can be a subject in a logical sentence are:
a) current day of the week
b) current clock time
c) geographic location of the user
d) financial status of the user
e) user's health condition
f) number of medications taken today
g) user's state of well-being
h) amount of energy burned in the last 24 hours

Examples of value types that can be a value in a logical sentence are:
a) a simple value: number, string, boolean value
b) a set of simple values: set of numbers, set of strings, set of boolean values

Examples of logical connectors between sentences A and B are:
a) A ∧ B - logical operation AND
b) A v B - logical operation OR

The user can assign a selected fitness configuration to their account. The user can view their fitness configuration in the client application at any time and trigger a physical activity analysis for the selected fitness configuration.

Physical activity analysis towards fitness configuration can be performed depending on the system operation mode: on the user device or on the server. The analysis process towards fitness configuration consists of the 3 phases:
a) downloading necessary data for analysis
b) first iteration of data processing
c) second iteration of data processing

The first phase is downloading necessary data for physical activity analysis. Regardless of where the analysis is processed (on the client device or on the server), the necessary data must first be downloaded. The minimum set of data needed is:
a) fitness configuration
b) physical activity description

Depending on your system settings and configuration settings, it may be possible or necessary to download additional data (for example: the user's history of physical activity over past few days, the user's health status, the user's geographic location, and more).

The next phase is the first iteration of data processing. For each regulation and tolerance defined in the fitness configuration, intermediate results are calculated. For each regulation, a conditions fulfillment result and a rules fulfillment result are determined. For each tolerance, a conditions fulfillment result are determined. In the case of an empty set of conditions, the entire regulation is always activated and calculations of the fulfillment of the rules are performed.

Determining the fulfillment of a single condition or rule comes down to checking the truth of a logical sentence. Example of condition or rule is:
a) dumbbell mass < 10 kg
b) today = first week of the month
c) activity required equipment list contains "bike"
d) amount of burned energy in total activity > 150 kcal

Each such logical sentence is calculated and the result is determined for 3 states:
a) success - sentence is true
b) fail - sentence is false
c) undetermined - the sentence is impossible to determine (e.g. the physical activity does not have the necessary data for analysis)

In the regulation, the results of conditions and rules are then calculated as a whole using logical connectors. For example for sentence A (which has state "success") and sentence B (which has state "undetermined"):
a) A ∧ B - result is false
b) A v B - result is true
c) B ∧ B - result is false
d) B v B - result is false

This means that for a set of sentences to be true, it must contain at least 1 true sentence. In this way, for each set of conditions and each set of rules, the result of fulfilling the set of sentences is calculated. At the end of this phase, each regulation and each tolerance has its own intermediate result of conditions and rules fulfillment.

The last phase of the analysis process is to determine the final result of the physical activity analysis. Iteration goes through all tolerances starting from the last one and ending with the first one. For each tolerance, the result of conditions fulfillment is checked. If the tolerance conditions are fulfilled, then this tolerance is activated. It means that the regulations and tolerances defined in this tolerance are being canceled from physical activity analysis. If the next tolerance on the list is "cancelled" it is not taken into account in the analysis and this tolerance is omitted. This means that the further on the list a tolerance is defined, the higher priority it has (because it can cancel the tolerances that are before it, unless it is canceled itself). After iterating through all tolerances and marking regulations and tolerances as canceled, the final physical activity analysis result is being calculated. Every regulation which has conditions fulfilled and is not canceled is taken into account in the analysis result.

The physical activity analysis result is calculated as the sum of the obtained values divided by the sum of the maximum possible "success" values of all active regulations. The result is truncated to a range of minimum 0% to maximum 100% so that the result value can be easily understood.

Provided that the result of the regulation may have not only a numerical value but also a special value:
a) full physical activity acceptance (final analysis result would be 100%)
b) full physical activity rejection (final analysis result would be 0%)

This means that it may happen that only 1 regulation out of many activated regulations will be decisive for awarding the maximum score (100%) or the minimum score (0%) for a given physical activity. For people with physically disabled legs it may be crucial to fully reject physical activities which require to use legs during activity (for example running).

The analysis result of physical activity or multiple physical activities is presented in the client application after the analysis is performed. The output file of the analysis towards fitness configuration contains:
a) final score (numerical value from 0% to 100%)
b) fitness configuration data along with intermediate results for each regulation and each tolerance

Fitness event is a file defining fitness parameters for a given activation factor. The basic activation factor is time trigger, but it can be any phenomenon (for example: user enters into a specific geographic area or notification on the user device about the user's current financial status). Fitness event is an abstract entity that defines the user's temporary fitness rules. Fitness event has 2 types of sentences:
a) regulation - a set of sentences that generates the component result of the analysis.
b) functional - a set of sentences that generates the component result of the analysis, but also can be used as sample input data for user.

Fitness event can be described by different type of event:
a) physical activity recommendation (e.g. gym workout, walking, rock climbing, swimming)
b) physical activity prohibition (e.g. all-day resting)

For example, a short fitness event (e.g. lasting 60 minutes) would typically determine features of a single moment of physical activity (e.g. one workout), whereas a long fitness event (e.g. lasting 7 days) would typically determine features of many different moments of physical activity (e.g. every fitness exercise for 7 days).

The regulations in the fitness event have the same structure as in the fitness configuration. They may, but do not have to, have a set of conditions. They must have a set of rules. The set of conditions contains logical sentences connected by logical connectors. The set of rules contains logical sentences connected by logical connectors. Each regulation has defined intermediate results for conditions fulfillment and rules fulfillment.

In addition, fitness event may contain functional sentences. A functional sentence comes down to applying the logic of "it is recommended to do" or "it is forbidden to do" for a specific entity. The entity may be a specific physical activity or a specific activity category. The entity may have defined value or value range. Examples of functional sentences are:
a) It is recommended to run 5 to 10 km (default 7 km)
b) It is forbidden to lift up more than 100 kg on the exercise bench

Each functional statement has its intermediate result for states identical to those in the case of regulation:
a) success - fulfilled sentence
b) fail - not fulfilled sentence
c) undetermined - the sentence is impossible to determine

In addition to its analytical function, a fitness event can act as an example or a suggestion for physical activity. If the event has specific physical activities defined, the user can treat them as a recommendation to consume those during that event. This way user can immediately go to the physical activity analysis towards fitness event using physical activities from this event.

The user can assign selected fitness events to the account. At any time in the client application, the user can view the list of fitness events and trigger the physical activity analysis for the selected fitness event. Additionally, the system can present a fitness event to the user once an activation factor is met. For example, if the activation factor is geographic area X and the user's position is monitored by the client application, then when the user enters geographic area X, the client application can present the desired fitness event.

The system can present fitness events sorted by the nearest activation factor to user. If fitness events are activated by time interval, the closest fitness events will be those with a time interval closest to the user's selected time. If fitness events are activated by geographic location, the closest fitness events will be those with the geographic location closest to the user's selected geographic location.

Physical activity analysis towards fitness event can be performed depending on the system operation mode: on the client device or on the server. The process of analyzing the fitness event consists of 2 phases:
a) download necessary data for analysis
b) first iteration of data processing

The first phase is downloading necessary data for physical activity analysis. Regardless of where the analysis is processed (on the client or on the server), the necessary data must first be downloaded. The minimum set of data needed is:
a) fitness configuration
b) physical activity description

Depending on your system settings and configuration settings, it may be possible or necessary to download additional data.

Next phase is to iteratively process the physical activity data towards fitness event regulation and obtain the final result of the physical activity analysis. Intermediate results are determined for each regulation and tolerance defined in the fitness event. For each regulation defined in the fitness event, intermediate results are calculated. For each regulation, a conditions fulfillment result and a rules fulfillment result are determined. In the case of an empty set of conditions, the entire regulation is always activated and calculations of the fulfillment of the rules are performed. Each functional sentence intermediate result is calculated and used in final analysis result.

The physical activity analysis result is calculated as the sum of the obtained values divided by the sum of the maximum possible "success" values of all active regulations and functional sentences. The result is truncated to a range of minimum 0% to maximum 100%.

The analysis result of physical activity or multiple physical activities is presented in the client application after the analysis is performed. The output file of the analysis towards fitness event contains:
a) final score (numerical value from 0% to 100%)
b) fitness event data along with intermediate results for each regulation and each functional sentence

### IMPLEMENTATION

The system can be used in many ways, both by an ordinary person in everyday life and by a specialist in their work:
1. A regular user can monitor and optimize their own physical activity - By using the system, the user consciously monitors physical activities. The user acquires knowledge about the effect a given physical activity has on the body. With this knowledge, user can optimize physical activity and respond appropriately to maintain the highest level of fitness and health (e.g. increasing physical activity after excessive energy consumption from food).
2. A regular user can monitor and optimize another person's physical activity (e.g. relative person with health problems or juvenile child) - Another important feature is the ability to analyze and monitor physical activities by someone else than yourself. This is important in the case of people who cannot or do not know how to use modern technological solutions on their own. It can be for example unwell relative, young child or elderly person. By analyzing the physical activities, fitness of this person can be optimized, ensuring better health.
3. A regular user can make conscious decisions about their physical activity knowing the personalized rating of each physical activity - The system can help make decisions about what the user should or should not exercise. Thanks to this, user can make better decisions in a store, restaurant or other place. This can result in a better financial situation for the user and help to minimize person overweight.
4. A sport trainer can monitor and optimize a person's physical activity - The system can facilitate and improve the quality of a trainer's work. The trainer can program the fitness configuration individually according to the needs of each person. Then, trainer can examine the person's physical activity and evaluate the fitness decisions they make.
5. A doctor in a medical facility (such as a hospital or clinic) can get to know patient's history of physical activity along with physical activity ratings - An additional advantage is the ability of the medical unit to retroactively analyze the physical activities by the patient who uses the system. In certain cases such as accident, illness or loss of consciousness, a person may not be able to explain their health condition, fitness needs and possibilities. In such situations, the doctor is dependent on information from third parties, which may be absent or the information may be incorrect. Thanks to the use of the system by the user, the doctor would have reliable information immediately. In many situations, this means a greater chance of saving the patient's health or life.

## Claims

1. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness configuration is stored on the user device; and the physical activity analysis is performed on the user device using the stored fitness configuration; and the physical activity analysis result is returned to the user device.

2. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness configuration is stored on the server; and the physical activity analysis is performed on the server using the stored fitness configuration; and the physical activity analysis result is returned to the user device.

3. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the user device; and the physical activity analysis is performed on the user device using the stored fitness event; and the physical activity analysis result is returned to the user device.

4. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the server; and the physical activity analysis is performed on the server using the stored fitness event; and the physical activity analysis result is returned to the user device.

5. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the user device; and the fitness event has an activation factor detectable on the user device; and the fitness event is activated on the user device; and the activated fitness event is returned to the user device.

6. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the server; and the fitness event has an activation factor detectable on the server; and the fitness event is activated on the server; and the activated fitness event is returned to the user device.

7. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the server; and the fitness event has an activation factor detectable on the user device; and the activation signal for fitness event is detected on the user device; and the activation signal for fitness event is send to the server; and the activated fitness event is returned to the user device.

8. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the user device; and the fitness event has an activation factor detectable by third party device or software; and the activation signal for fitness event is detected; and the activation signal for fitness event is send to user device; and the activated fitness event is returned to the user device.

9. A system comprising a user device, a server, and a connection between them. The user device comprises a processor, input-output devices, operating memory, and persistent memory. The server comprises a processor, input-output devices, operating memory, and persistent memory. The user device runs a client application. The server runs a server application. The system, wherein the fitness event is stored on the server; and the fitness event has an activation factor detectable by third party device or software; and the activation signal for fitness event is detected; and the activation signal for fitness event is send to the server; and the activated fitness event is returned to the user device.

10. The system of claim 2, wherein the physical activity analysis is using physical activity data received from server.

11. The system of claim 4, wherein the physical activity analysis is using physical activity data received from server.

12. The system of claim 2, wherein the physical activity analysis is using physical activity data received from third party.

13. The system of claim 4, wherein the physical activity analysis is using physical activity data received from third party.
